# EUROPEAN PATENT SPECIFICATION

(11) **EP 3 988 238 B1**
(45) Date of publication and mention of the grant of the patent: **14.08.2024**
(21) Application number: 21204058.8
(22) Date of filing: 21.10.2021
(51) Int. Cl.: B23K 31/00, B23K 9/32

(54) **WELDING SHIELD WITH ARTIFICIAL INTELLIGENCE-CONTROLLED DISPLAY**
SCHWEISSSCHUTZSCHILD MIT DURCH KÜNSTLICHE INTELLIGENZ GESTEUERTER ANZEIGE
MASQUE DE SOUDAGE AVEC AFFICHAGE CONTRÔLÉ PAR INTELLIGENCE ARTIFICIELLE

(30) Priority: 24.10.2020 RU 2020134919; 07.10.2021 US 202117496621
(43) Date of publication of application: 27.04.2022
(73) Proprietor: Malov, Alexey, Saint-Petersburg 193231 (RU)
(72) Inventor: Malov, Alexey, Saint-Petersburg 193231 (RU)
(74) Representative: Leach, Sean Adam

(56) References cited:
- WO-A1-2016/144744
- US-A1- 2012 291 172
- US-A1- 2016 207 134
- US-A1- 2017 227 766
- PARK JE-KANG ET AL: "Convolutional Neural Network Based Surface Inspection System for Non-patterned Welding Defects", INTERNATIONAL JOURNAL OF PRECISION ENGINEERING AND MANUFACTURING, KOREAN SOCIETY FOR PRECISION ENGINEERING, SPRINGER, vol. 20, no. 3, 22 February 2019 (2019-02-22), pages 363 - 374, XP036741899, ISSN: 2234-7593, [retrieved on 20190222], DOI: 10.1007/S12541-019-00074-4

## Description

### TECHNICAL FIELD

The present disclosure relates to the field of welding production and can be used to protect a person during welding. More specifically, the present disclosure relates to personal protective equipment that has artificial intelligence.

### BACKGROUND OF THE RELATED ART

A curved auto-darkening light filter (RU patent No. 2719306, priority dated 12/13/2012) is known. The light filter changes its state from light-transmitting to shade mode under the influence of incident light. The auto-darkening light filter is manufactured by a method based on forming a liquid crystal cell. The invention provides a reduction in weight and an expansion of the field of view for the user.

The disadvantages of welding shields based on this device, as well as other well-known commercial welding shields with auto-darkening light filters (lincolnelectric.com/en/Products/Safety/Head-Face-and-Eye/Welding-Helmets-and-Accessories/VIKING-3350-Series) are listed below:

When voltaic arc light is detected, the light filter enters shade mode for protection the welder from dazzle. In this mode voltaic arc area and a small area around it remains light, but not dazzling, and the rest of the area is darkened. Thus, only a small area voltaic arc the arc is clearly visible. The welder does not see everything that is around. In such case, for example, it is difficult to estimate when the end of the soldering line will be reached, what part of the soldering line is done. You need to rely on visual memory and evaluate intuitively, or temporarily stop welding to get the filters out of shade mode.

Also, in a shade mode, the arc area and a small area around it remains light, while the rest are darkened. Such a regime is unnatural for the eyes and leads to increased fatigue.

The closest technical solution is a shield for welding (RU patent No. 2457815, priority dated 04/05/2011), which contains an actuator attached to it, a viewing slot and a holder with a light filter installed with the ability to close and open the slot by means of a mechanism, a spring, mouthpiece and air duct. The end of the mouthpiece is located near the lips. The actuator is made in the form of a bellows with a stem. The air duct is fixedly fixed on the faceplate and is connected at one end to the mouthpiece, and at the other end to the bellows, the stem of which is connected to the light filter holder rotating around the axis. The spring is fixed on the holder and face shield.

The disadvantages of this shield include the disadvantages listed for the invention described previously. Another disadvantage is the need for the welder to control the movement of the light filter using the pressure in the mouth.

US 2017/227766 A1 describes methods and systems for control of mediated reality welding systems based on lighting conditions. US 2016/207134 A1 describes methods and systems for weld output control by a welding vision system. US 2012/291172 A1 relates to the visualization of arc welding characteristics during an arc welding process.WO 2016/144744 A1 describes methods and systems for networked high dynamic range welding vision system. Park Je-Kang et al: "Convolutional Neural Network Based Surface Inspection System for Non-patterned Welding Defects" (2019) describes a CNN based method that inspects non-patterned welding defects on the surface of the engine transmission using a single RGB camera.

### SUMMARY OF THE INVENTION

In one aspect, there is provided a welder's shield with artificial intelligence including a frame for mounting on a user's head, a camera that obtains images of a welding zone and providing video data to a processor, a display on an inner side of the frame, an optical device to enable the user to see the display and an image processing application running on the processor for analyzing the video data from the video camera, the processor displaying images on the display based on an output of the image processing application, the image processing application detecting a welding arc in the welding zone using a pattern recognition algorithm and the image processing application modifying the video data to reduce an intensity of the welding arc in the images that are to be shown on the display, without reducing an intensity of a remainder of objects in the images being displayed. Optionally, the welder's shield comprises a frame for mounting on a user's head; a camera mounted on the frame and directed forward toward a welding zone, so as to obtain images of the welding zone as video data; a display on an inner side of the frame; an optical device to enable the user to see the display; a processor coupled to a memory; a battery providing power to the processor, the display and the camera; the processor receiving a video data from the camera; the processor running an artificial intelligence application to process the video data from the video camera; the processor displaying images on the display based on output of the artificial intelligence application; the artificial intelligence application receiving the video data and detecting a welding arc in the welding zone using a pattern recognition algorithm; and the artificial intelligence application modifying the video data to reduce an intensity of the welding arc in the images that are to be displayed on the display, without reducing an intensity of a remainder of objects in the images being displayed.

Optionally, the video data is provided from the video camera to the processor using a wireless interface. Optionally, the video data is provided from the video camera to the processor using a high definition multimedia wire interface. Optionally, the video data is provided from the processor to the display using a high definition multimedia wire interface. Optionally, the video data is provided to the processor using a universal serial bus wire interface. Optionally, the artificial intelligence application processes the video data on a frame by frame basis. Optionally, the pattern recognition algorithm uses a histogram of oriented gradients for detecting the welding arc. Optionally, the pattern recognition algorithm uses a convolutional neural network for detecting the welding arc. Optionally, the recognition algorithm uses prepared pre-trained model based on neural network or histogram of oriented gradients, and wherein the model has an object corresponding to the welding arc. Optionally, the artificial intelligence application provides for adjustment of a photosensitivity of the video camera based on a brightness of the welding arc. Optionally, the artificial intelligence application provides for adjustment of a photosensitivity of the video camera based on a brightness of a surrounding environment. Optionally, a graphical processing unit is coupled to the processor for processing the images.

Additional features and advantages of the invention will be set forth in the description that follows, and in part will be apparent from the description, or may be learned by practice of the invention. The advantages of the invention will be realized and attained by the structure particularly pointed out in the written description and claims hereof as well as the appended drawings.

It is to be understood that both the foregoing general description and the following detailed description are exemplary and explanatory and are intended to provide further explanation of the invention as claimed.

### BRIEF DESCRIPTION OF THE ATTACHED FIGURES

The accompanying drawings, which are included to provide a further understanding of the invention and are incorporated in and constitute a part of this specification, illustrate embodiments of the invention and, together with the description, serve to explain the principles of the invention.

In the drawings:
**FIG. 1** illustrates a welding shield that includes a power source, digital video cameras, display and computing device, in accordance with the invention.
**FIG. 2** is an exemplary system architecture.
**FIG. 3** is a flow chart associated with the operation algorithm of the welder's shield in accordance with the invention.

### DETAILED DESCRIPTION OF THE PREFERRED EMBODIMENTS

Reference will now be made in detail to the preferred embodiments of the present invention, examples of which are illustrated in the accompanying drawings.

The technical result is an improvement in working conditions, an increase in work productivity.

The task is solved as follows. The welding shield is a head-mounted device comprising:
(1) autonomous power source, for example, an accumulator battery
(2) One or several digital video cameras on the outer side of the shield. Above-mentioned digital video cameras directed in the direction of the welder's direct gaze, allowing to obtain an image with an angle of coverage of at least a direct view of the welder. Digital video cameras above designed for stable operation in the presence of bright light sources.
(3) display on the inside side of the shield
(4) an optical system located in front of the display and allowing the welder to adjust the image to the individual characteristics of the welder's vision
(4) a computing device comprising permanent storage device, random access memory, one or more computer processors, and an installed general-purpose operating system. Also, the data computing device has interfaces to which the above-mentioned video cameras and display are connected. The computing device also contains installed and configured software capable of:
   (a) to receive data from above-mentioned video cameras
   (b) to run an artificial intelligence application on execution to process data from video cameras
   (c) to send data from video cameras to an artificial intelligence application
   (d) to display data received from an artificial intelligence application on the display
(4) an artificial intelligence application running on an above-mentioned computing device capable of:
   (a) to receive video data received from video cameras
   (b) to detect the appearance of a welding arc using pattern recognition algorithms, change the resulting image and instead of the welding arc put instead of the welding arc an illuminated spot that is comfortable for the eye
   (c) to send the processed image for display
   (d) to provide processing of video data with a delay visually imperceptible to humans
   (e) to provide adjustment of the video camera's light sensitivity based not on the brightness of the arc, but on the brightness comfortable for the welder's eye

From the prior art, no solutions have been identified that have features that coincide with the distinctive features of the claimed invention and have the same effect as they do on the technical result, which consists in improving working conditions, increasing labor productivity. These distinctive features allow achieving the following advantages over the prototype:
(1) Not only a small area around the welding arc is clearly visible, but the entire area of the line of sight.
(2) The entire screen is evenly illuminated and not one bright spot in the welding arc area and a small area nearby. This mode is natural for the organs of vision and reduces fatigue.
(3) There is no need for the welder to control the movement of the filter using the pressure in the mouth.

The welder's shield works as follows: after switching on the device, one or several digital video cameras on the outside of the shield make video and continuously sends the image to the computing device. The computing device and the software running on it send the video image frame by frame to the artificial intelligence application. An artificial intelligence application processes each frame using pattern recognition algorithms, identifying the welding arc if present. Changes the resulting image, replaces the welding arc with an illuminated spot that is comfortable for the eye. Comfort is explained by the fact that a uniform distribution of brightness in the field of view is essential for human working capacity. [Alekseev S.V., Usenko V.R. Gigiena truda. Moscow: Medicina, 1988. Glava 19. URL: nashaucheba.ru/v2562/alekseev_s.v.,usenko_v.r._gigiena_truda?page=22 (Accessed October 24, 2020)]

At the same time, it is possible to use digital light filters and additional darkening of the overly illuminated area around the welding arc to a level of average illumination in the field of vision, which is comfortable for the welder's eyes. If necessary, the artificial intelligence application provides adjustment of the brightness sensitivity of video cameras, based not on the brightness of the arc, but on a level of average illumination in the field of vision, to form an image of the desired brightness. The welder can adjust the brightness of the entire display to the individual characteristics of the welder's vision using, for example, the adjustment buttons on the welding shield. Further, the artificial intelligence application sends the resulting image through the computing device and the interfaces connected to it to the display for output. The artificial intelligence application could perform this without the usage of artificial intelligence algorithms, but rather by using graphical libraries, providing a wide set of features for working with images, such as the OpenCV library [pypi.org/project/opencv-python/].

Thus, unlike the prototype and known technical solutions, after turning on the welder's shield, the welder always sees a uniformly illuminated screen. As mentioned above, the uniform distribution of brightness in the field of view is essential for human working capacity. [Alekseev S.V., supra] This opportunity improves working conditions. There is no need for the welder to control the movement of the filter using the pressure in the mouth. This feature automatically protects the vision from overloading when a welding arc occurs. Not only a small area around the electric arc is clearly visible, but the entire area of the line of sight. The welder does not need to be interrupted to inspect the area to be welded, to estimate when the end of the soldering line will be reached, what part of the soldering line is done. You don't need to rely on visual memory and evaluate intuitively. These features help to increase labor productivity.

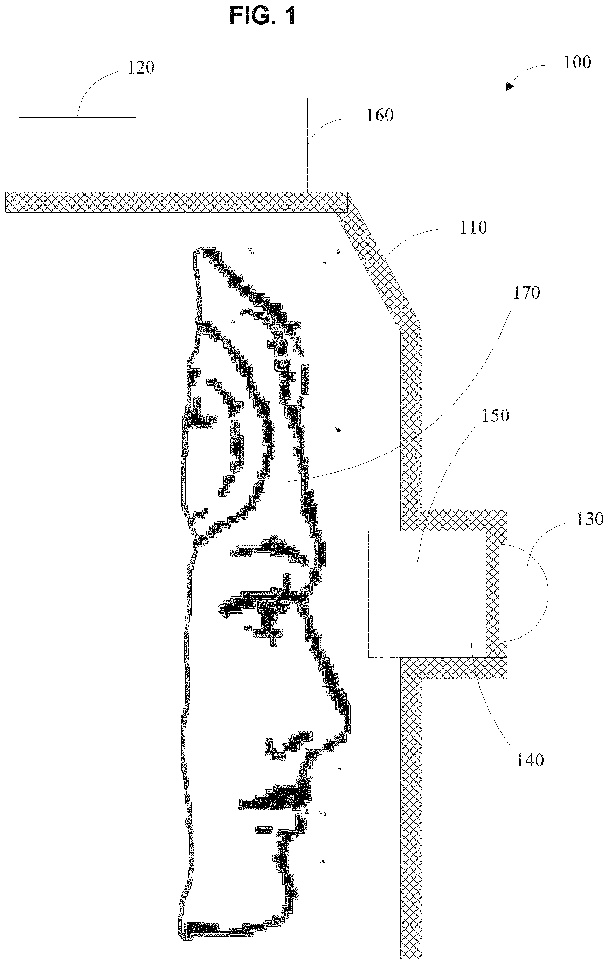

**FIG. 1** illustrates a welding shield **100.** The system of welding shield includes the frame of the welding shield **110.** Frame of the welding shield is fixed on the head of a welder **170.**

A power source **120** and computing device **160** are attached to the top of the frame of the welding shield. One or more video cameras **130** are located on the outside of the welder's shield. These video cameras must be resistant to bright light and ultraviolet radiation. Display **140** is located on the inside of the shield in front of the welder's eyes.

An optical system **150** is located on the inside of the shield in front of the display **140.** The system allows the welder to adjust the image to the individual characteristics of the welder's vision. It includes two regulators. One regulator allows you to adjust the setting to take into account myopia, hyperopia in both eyes. The second regulator allows you to adjust the setting for the characteristics of one of the eyes, if the eyes have different visual characteristics.

The computing device contains software including a real-time operating system. The real-time operating system ensures that the latency does not exceed a certain amount of time. WindRiver Linux [windriver.com/products/linux/] can be used as a real-time operating system.

The task of detecting images belongs to the field of artificial intelligence. The application operating in the device that detects the occurrence of a welding arc is an artificial intelligence application. There are ready-made libraries that allow you to detect images in applications. The artificial intelligence application can use Mask R-CNN machine learning detector library [https://github.com/matterport/Mask_RCNN] to detect the appearance of a welding arc. Such libraries can use machine learning models based, for example, on neural networks or histogram of oriented gradients, depending on the implementation. The detection functionality of Mask R-CNN library is based on convolutional neural network with some additional features for higher performance.

Common Objects In Context dataset [cocodataset.org/#home] is suitable as a training dataset for training a model based on Mask R-CNN. This training set should be extended with marked welding arc images. Also, a new object type should be added to the training dataset for training a model corresponding to the welding arc. In this case, a pre-trained model should be prepared based on the Mask R-CNN with the above dataset. Prepared pre-trained model should be copied to the permanent storage device of the computing device. This model will be used by an artificial intelligence application for detect a welding arc by means of Mask R-CNN library.

An artificial intelligence application can use the OpenCV library [pypi.org/project/opencv-python/] to change the resulting image and replace the welding arc with a bright spot that is comfortable for the eye.

Mask R-CNN model returns four items for each recognized object on the frame:
(1) The integer value of the type of the detected object.
(2) The degree of confidence in the recognition results. The higher the number, the more confident the model is in recognizing the object correctly.
(3) A bounding box for an object in the form of the XY coordinates of pixels in the frame.
(4) A mask which indicates which pixels within the bounding box are part of the object. Using the mask, one can find the outline of an object.

Below is the example of the code for detecting welding arcs using the Mask R-CNN pre-trained model. The notation of Python programming language will be used in example [***.python.org].

```
 import mrcnn.config
 import mrcnn.utils
 from mrcnn.model import MaskRCNN
 # the importing of all other required libraries
 # a class setting configuration of Mask-RCNN library.
 class MaskRCNN_config(mrcnn.config.Config):
           NAME = " pretrained_model_with_welding_arc_config"
           IMAGES_PER_GPU =... #required value
           GPU_COUNT =... #required value
           NUM_CLASSES = ... #required value
           DETECTION MIN CONFIDENCE = ... #required value
  
 # Initialize Mask R-CNN model.
 model_instance=MaskRCNN(mode="inference",model_dir=MODEL_DIR,config=MaskRC
 NN_config())
  
 # Load Mask R-CNN pre-trained model from permanent storage device.
 model.load_weights(COCO_MODEL_PATH, by_name=True)
  
 def check_frame_for_welding_arc():
      #obtain current frame
      cur_frame = ...
      # Detecting objects in the frame
      results = model_instance.detect([cur_frame], verbose=0)
      # Mask R-CNN assumes that objects in multiple images are recognized
      # One image passed in this example, so it's need to retrieve only the first result.
      first_result = results[0]
      # The variable first_result now contains the detection results and has next fields:
      # - first_result ['rois'] - a bounding box for each object;
      # - first_result ['class_ids'] - the integer value of the type for each object;
      # - first_result ['scores'] - the degree of confidence for each object;
      # - first_result ['masks'] - a mask for each object.
  
      for cur_index, cur_box in enumerate(first_result ['rois']):
           # Check if current object is welding arc
           if class _ids[cur _index] == WeldingArcClassId:
                #Welding arc found handle this case
                handle_weldeing_arc_detection()
```

In the example above the function MaskRCNNConfig used for set required parameters for Mask R-CNN model. In the example the class instance model_instance is Mask R-CNN model. The function check_frame_for_welding_arc illustrates the way to detect a welding arc with usage of Mask R-CNN and other above-mentioned libraries. The function check_frame_for_welding_arc obtains current frame for handling in the variable cur_frame by some way omitted in the example for simplicity. The call of the function model_instance.detect allows get all detected object in the variable first_result. After the find of welding arc is performed in the cycle throw all detected objects. If welding arc is found, the function handle_welding_arc_detection is called.

It should be noted that in some embdiments the computing device and all its components, including permanent storage device, random access memory, one or more computer processors, and interfaces have sufficient hardware performance to ensure processing and transmission of video data with a delay visually imperceptible for the welder. Mask R-CNN architecture is designed in such a way that object identification is quite fast. With a modern graphical processing unit (GPU), one can identify objects in high-definition video at a speed of several frames per second. The advantages of the GPU in the performance of objects detection appear when processing high-resolution images. (***.researchgate.net/publication/342575655_CPU_vs_GPU_performance_of_deep_learnin g_based_face_detectors_using_resized_images_in_forensic_applications) This should be sufficient for these tasks. Thus, the computing device may have a high performance graphical processing unit for fast image processing and identification.

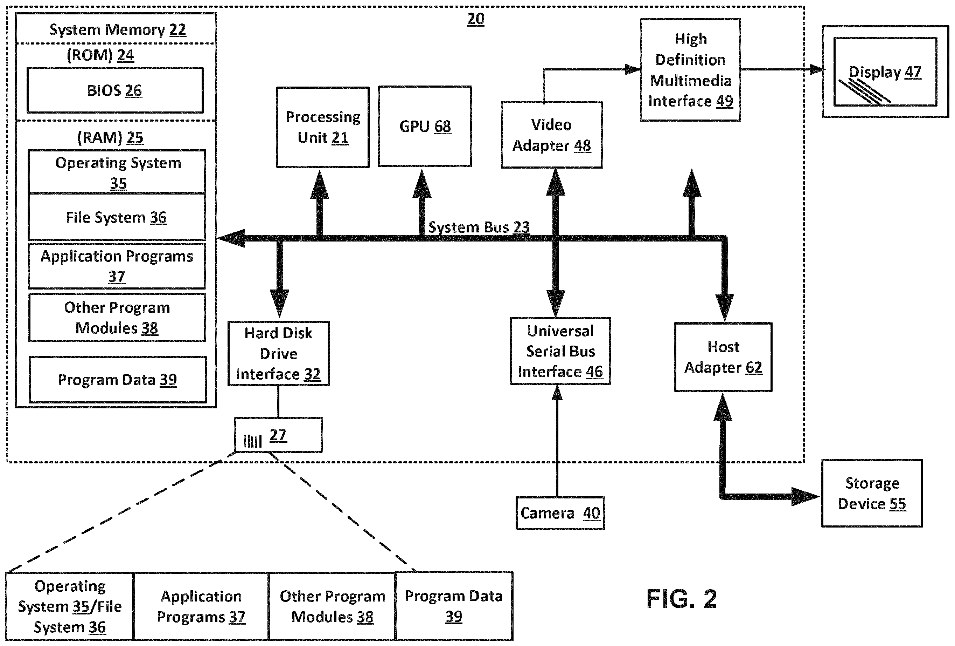

With reference to **FIG. 2****,** an exemplary system for implementing the invention includes a general purpose computing device in the form of a host computer **20** or the like, including a processing unit (CPU) **21,** one or more Graphical Processing Units **68,** a system memory **22,** and a system bus **23** that couples various system components including the system memory to the processing unit **21.**

The system bus **23** may be any of several types of bus structures including a memory bus or memory controller, a peripheral bus, and a local bus using any of a variety of bus architectures. The system memory includes a read-only memory (ROM) **24** and random access memory (RAM) **25.** A basic input/output system **26** (BIOS), containing the basic routines that help to transfer information between the elements within the computer **20,** such as during start-up, is stored in ROM **24.**

The computing device **20** may further include a hard disk drive **27** for reading from and writing to a hard disk, not shown herein. The hard disk drive **27** is connected to the system bus **23** by a hard disk drive interface **32.**

The drives and their associated computer-readable media provide non-volatile storage of computer readable instructions, data structures, program modules and other data for the computing device **20.** Although the exemplary environment described herein employs a hard disk (storage device **55**).

A number of program modules may be stored on the hard disk (storage device **55**), ROM **24** or RAM **25,** including an operating system **35** (e.g., Android, LINUX or similar). The computing device **20** includes a file system **36** associated with or included within the operating system **35,** such as the fourth extended file system (ext4fs) or similar, one or more application programs **37,** other program modules **38** and program data **39.** A user may switch on/of the computing device **20** with usage of power button (not shown). A host adapter **62** is used to connect to the storage device **55.**

A digital video camera **40** and other input devices are often connected to the processing unit **21** through a universal serial bus (USB) interface **46** that is coupled to the system bus, and they may also be connected by other interfaces. A display **47** is also connected to the system bus **23** via a video adapter **48** and an interface, such as a high definition multimedia interface (HDMI) **49.** Also, the digital video camera **40** can be connected to the processing unit **21** through a wireless interface, for example, Wi-Fi or BLUETOOTH. Wi-Fi interface use to transmit a data by air. After receiving data by air by means of Wi-Fi interface a data are usually send to the wired interface like HDMI. An example is wireless screen mirroring receiver [any-cast.com/blogs/user-manual/m100-user-manual].

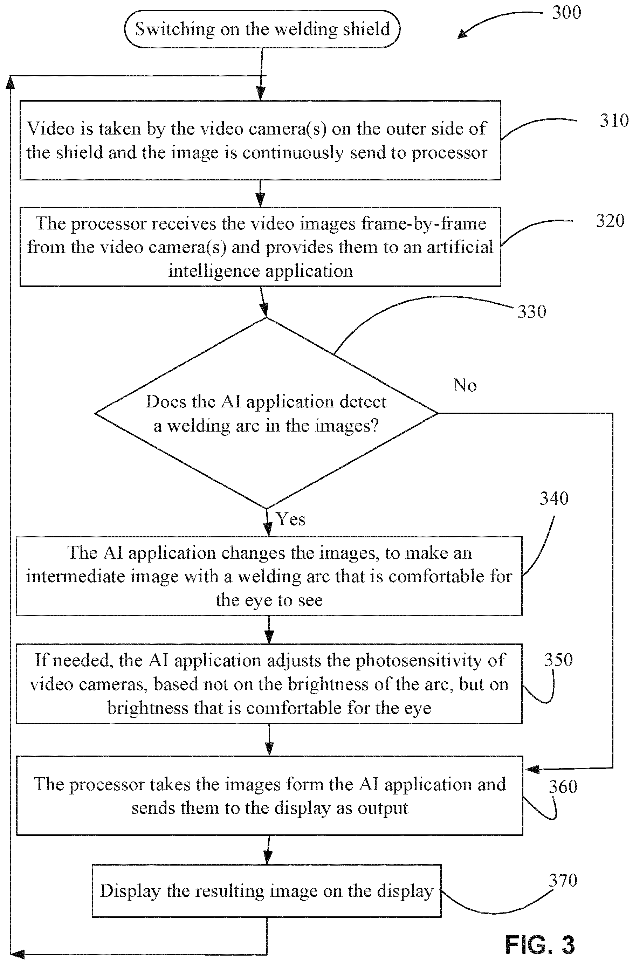

**FIG. 3** is a flow chart **300** associated with the operation algorithm of the welder's shield. While the steps shown in **FIG. 3** are exemplary operations associated with the present disclosure, variations on the order of the steps, and additional steps, will be apparent to one of skill in the art upon reading the present disclosure.

After switching on the welder's shield video is taken by means of one or more of the mentioned-above video cameras on the outer side of the shield and the image is continuously send to computing device via the mentioned-above interfaces (**310**) continuously. Further by means of a computing device and software operating on it, video images are received frame-by-frame from mentioned-above one or more video cameras and send to an artificial intelligence application (**320**). By means of an artificial intelligence application, each video frame is processed using pattern recognition algorithms, and the welding arc is detected if there is one (**330**). If a welding arc is detected in step **330,** by means of an artificial intelligence application, the resulting image is changed, put instead of the welding arc an illuminated spot that is comfortable for the eye, and make by this way an intermediate image (**340**). Also if necessary, by means of an artificial intelligence application, they provide adjustment of the photosensitivity of video cameras, based, for example, not on the brightness of the arc, but on the brightness that is comfortable for the welder's eye, to form an image of the desired brightness (**350**). Another option is to adjust the photosensitivity of the camera based on a brightness of the surrounding environment. Comfort is explained by the fact that a uniform distribution of brightness in the field of view is essential for human working capacity. [Alekseev S.V., Usenko V.R. Gigiena truda. Moscow: Medicina, 1988. Glava 19. URL: nashaucheba.ru/v2562/alekseev_s.v.,usenko_v.r._gigiena_truda?page=22 (Accessed October 24, 2020)]

By means of an artificial intelligence application, the resulting image is sent through the computing device and the interfaces connected to it to the mentioned-above display for output (**360**). Step **360** is performed in any case, whether welding arc was detected or not in step **330.** At the end of operation cycle device displays the resulting image on the display (**370**). After step **370** the operation algorithm of the welder's shield moves cyclically to the first step **310** to handle the next video frame.

Also, before sending the processed image to the display in step **360,** the artificial intelligence application can combine the data received from infrared and video cameras, and generate using the OpenCV library the resulting image with the highlighting of hot areas with a special range of colors. In this case, information about the temperature comes from one or more infrared cameras. Special colors that characterize the surface temperature are superimposed on the video image before being sent to the display on the inside of the shield. Also, for example, a color scale can be located in the corner of the resulting image, showing the approximate correspondence of color and temperature in the welding area. If there are several digital video cameras in the device, the artificial intelligence application can combine several frames from different video cameras into one resulting image, with goal to obtain advanced characteristics of the result image.

The display can show the battery charge level, the remaining battery time, the current time. A flashing indicator, for example, green, can be displayed in the corner of the display, which signals the correct operation of the device.

The brightness which is comfortable for the wearer's eye may comprise a selected brightness level, for example selected based on the brightness of parts of the image other than the detected arc. The selected brightness level may be predetermined, for example and stored in a memory of the device. The welder can adjust the brightness of the entire display to the individual characteristics of the welder's vision using, for example, the adjustment buttons on the welding shield.

Additional features and advantages of the invention will be apparent from the description, or may be learned by practice of the invention. The advantages of the invention will be realized and attained by the structure particularly pointed out in the written description and claims hereof as well as the appended drawings.

Having thus described a preferred embodiment, it should be apparent to those skilled in the art that certain advantages of the described apparatus have been achieved. It should also be appreciated that various modifications, adaptations, and alternative embodiments thereof may be made within the scope of the present invention, as defined by the appended claims.

## Claims

1. A welder's shield (100) with artificial intelligence comprising:
a frame (110) for mounting on a user's head;
a camera (130) that obtains images of a welding zone and providing video data to a processor (160);
a display (140) on an inner side of the frame;
an optical device (150) to enable the user to see the display; and
an image processing application running on the processor for analyzing the video data from the video camera;
the processor displaying images on the display based on an output of the image processing application;
the welder's shield **characterized by**:
the image processing application detecting a welding arc in the welding zone using a pattern recognition algorithm; and
the image processing application modifying the video data to reduce an intensity of the welding arc in the images that are to be shown on the display, without reducing an intensity of a remainder of objects in the images being displayed.

2. The shield of claim 1, wherein:
the camera is mounted on the frame and directed forward toward the welding zone, so as to obtain the images of the welding zone as the video data;
the processor is coupled to a memory;
the shield further comprising a battery providing power to the processor, the display and the camera;
the processor running an artificial intelligence application to process the video data from the video camera;
the processor displaying images on the display based on output of the artificial intelligence application;
the artificial intelligence application receiving the video data and detecting a welding arc in the welding zone using a pattern recognition algorithm; and
the artificial intelligence application modifying the video data to reduce an intensity of the welding arc in the images that are to be displayed on the display, without reducing an intensity of a remainder of objects in the images being displayed.

3. The shield of claim 2, wherein the video data is provided from the video camera to the processor using a wireless interface.

4. The shield of claim 2, wherein the video data is provided from the video camera to the processor using a high definition multimedia wire interface.

5. The shield of claim 2, wherein the video data is provided from the processor to the display using a high definition multimedia wire interface.

6. The shield of claim 2, wherein the video data is provided to the processor using a universal serial bus wire interface.

7. The shield of any of claims 2-6, wherein the artificial intelligence application processes the video data on a frame by frame basis.

8. The shield of any preceding claim, wherein the pattern recognition algorithm uses a histogram of oriented gradients for detecting the welding arc.

9. The shield of any preceding claim, wherein the pattern recognition algorithm uses a convolutional neural network for detecting the welding arc.

10. The shield of claim 1, wherein the pattern recognition algorithm uses prepared pre-trained model based on neural network or histogram of oriented gradients, and wherein the model has an object corresponding to the welding arc.

11. The shield of any of claims 2-10, wherein the artificial intelligence application provides for adjustment of a photosensitivity of the video camera based on a brightness of the welding arc.

12. The shield of any of claims 2-11, wherein the artificial intelligence application provides for adjustment of a photosensitivity of the video camera based on a brightness of a surrounding environment.

13. The shield of claim 1, further comprising a graphical processing unit coupled to the processor for processing the images.

14. The shield of claim 1, wherein the image processing application uses a convolutional neural network for detecting the welding arc.

15. The shield of claim 1, wherein the image processing application uses prepared pre-trained model based on neural network or histogram of oriented gradients, and wherein the model has an object corresponding to the welding arc.

## Patentansprüche

1. Ein Schweissschutzschild (100) mit Künstlicher Intelligenz besteht aus:
einen Rahmen (110) zur Befestigung am Kopf eines Benutzers;
eine Kamera (130), die Bilder von einer Schweißzone aufnimmt und Videodaten an einen Prozessor (160) übermittelt;
einen Bildschirm (140) an einer Innenseite des Rahmens;
ein optisches Gerät (150), das es dem Benutzer ermöglicht, den Bildschirm zu sehen; und
eine Bildverarbeitungsanwendung, die auf dem Prozessor läuft, um die Videodaten von der Videokamera zu analysieren;
der Prozessor zeigt Bilder auf dem Bildschirm an, die auf einer Ausgabe der Bildverarbeitungsanwendung basieren.
Das Schweissschutzschild ist **gekennzeichnet durch**:
die Bildverarbeitungsanwendung erkennt einen Schweißbogen in der Schweißzone unter Verwendung eines Mustererkennungsalgorithmus; und
die Bildverarbeitungsanwendung modifiziert die Videodaten, um die Intensität des Schweißbogens in den Bildern, die auf dem Bildschirm angezeigt werden sollen, zu reduzieren, ohne die Intensität der restlichen Objekte in den dargestellten Bildern zu verringern.

2. Der Schild nach Anspruch 1, wobei:
die Kamera ist am Rahmen befestigt und nach vorne auf die Schweißzone gerichtet, um die Bilder der Schweißzone als Videodaten zu erhalten;
der Prozessor ist mit einem Speicher verbunden;
der Schild umfasst außerdem eine Batterie, die den Prozessor, den Bildschirm und die Kamera mit Strom versorgt;
der Prozessor, auf dem eine Anwendung der Künstlichen Intelligenz läuft, um die Videodaten der Videokamera zu verarbeiten;
der Prozessor zeigt Bilder auf dem Bildschirm an, die auf der Ausgabe von der Anwendung der Künstlichen Intelligenz basieren;
die Anwendung der Künstlichen Intelligenz die Videodaten empfängt und einen Schweißbogen in der Schweißzone unter Verwendung eines Mustererkennungsalgorithmus erkennt; und
die Anwendung der Künstlichen Intelligenz modifiziert die Videodaten, um die Intensität des Schweißbogens in den Bildern, die auf dem Bildschirm angezeigt werden sollen, zu reduzieren, ohne die Intensität der restlichen Objekte in den dargestellten Bildern zu verringern.

3. Der Schild nach Anspruch 2, wobei die Videodaten von der Videokamera über eine drahtlose Schnittstelle an den Prozessor übermittelt werden.

4. Der Schild nach Anspruch 2, wobei die Videodaten von der Videokamera über eine hochauflösende Multimedia-Drahtschnittstelle an den Prozessor übermittelt werden.

5. Der Schild nach Anspruch 2, wobei die Videodaten vom Prozessor über eine hochauflösende Multimedia-Drahtschnittstelle an den Bildschirm übermittelt werden.

6. Der Schild nach Anspruch 2, wobei die Videodaten dem Prozessor über eine universelle serielle Busschnittstelle übermittelt werden.

7. Der Schild nach einem der Ansprüche 2-6, wobei die Anwendung der Künstlichen Intelligenz die Videodaten Bild für Bild verarbeitet.

8. Der Schild nach einem der vorhergehenden Ansprüche, wobei der Mustererkennungsalgorithmus ein Histogramm von orientierten Gradienten zur Erkennung des Schweißbogens verwendet.

9. Der Schild nach einem der vorhergehenden Ansprüche, wobei der Mustererkennungsalgorithmus ein neuronales Faltungsnetzwerk zur Erkennung des Schweißbogens verwendet.

10. Der Schild nach Anspruch 1, wobei der Mustererkennungsalgorithmus ein vorbereitetes, vortrainiertes Modell auf der Grundlage eines neuronalen Netzes oder eines Histogramms orientierter Gradienten verwendet, und wobei das Modell ein Objekt enthält, das dem Schweißlichtbogen entspricht.

11. Der Schild nach einem der Ansprüche 2-10, wobei die Anwendung der Künstlichen Intelligenz die Einstellung der Lichtempfindlichkeit der Videokamera auf der Grundlage der Helligkeit des Schweißbogens ermöglicht.

12. Der Schild nach einem der Ansprüche 2-11, wobei die Anwendung der Künstlichen Intelligenz die Einstellung der Lichtempfindlichkeit der Videokamera auf der Grundlage der Helligkeit der Umgebung ermöglicht.

13. Der Schild nach Anspruch 1 umfasst außerdem einen mit dem Prozessor verbundenen Grafikprozessor zur Verarbeitung der Bilder.

14. Der Schild nach Anspruch 1, wobei die Bildverarbeitungsanwendung ein neuronales Faltungsnetzwerk zur Erkennung des Schweißbogens verwendet.

15. Der Schild nach Anspruch 1, wobei die Bildverarbeitungsanwendung ein vorbereitetes, vortrainiertes Modell verwendet, das auf einem neuronalen Netz oder einem Histogramm orientierter Gradienten basiert, und wobei das Modell ein Objekt enthält, das dem Schweißbogen entspricht.

## Revendications

1. Un masque de soudage (100) avec intelligence artificielle **caractérisé en ce qu'**il comporte :
un cadre (110) pour être monté sur la tête de l'utilisateur ;
une caméra (130) qui obtient des images d'une zone de soudage et fournit des données vidéo à un processeur (160) ;
un écran (140) sur le côté intérieur du cadre ;
un dispositif optique (150) permettant à l'utilisateur de voir l'écran ;
une application de traitement d'images fonctionnant sur le processeur pour analyser les données vidéo provenant de la caméra ;
le processeur affichant des images sur l'écran en fonction d'une sortie de l'application de traitement d'images ;
le masque de soudage **caractérisé en ce que** :
l'application de traitement d'images détecte un arc de soudage dans la zone de soudage à l'aide d'un algorithme de reconnaissance de formes ;
l'application de traitement d'images modifie les données vidéo pour réduire l'intensité de l'arc de soudage dans les images à afficher sur l'écran, sans réduire l'intensité des autres objets dans les images affichées.

2. Masque selon la revendication 1 **caractérisé en ce que** :
la caméra est montée sur le cadre et orientée vers l'avant en direction de la zone de soudage, de manière à obtenir les images de la zone de soudage en tant que données vidéo ;
le processeur est couplé à une mémoire ;
le masque comprenant en outre une batterie fournissant de l'énergie au processeur, à l'écran et à la caméra ;
le processeur exécute une application d'intelligence artificielle pour traiter les données vidéo provenant de la caméra ;
le processeur affiche des images sur l'écran en fonction de la sortie de l'application d'intelligence artificielle ;
l'application d'intelligence artificielle reçoit les données vidéo et détecte un arc de soudage dans la zone de soudage à l'aide d'un algorithme de reconnaissance de formes ;
l'application d'intelligence artificielle modifie les données vidéo pour réduire l'intensité de l'arc de soudage dans les images à afficher sur l'écran, sans réduire l'intensité des autres objets dans les images affichées.

3. Masque selon la revendication 2 **caractérisé en ce que** les données vidéo sont fournies par la caméra au processeur via une interface sans fil.

4. Masque selon la revendication 2 **caractérisé en ce que** les données vidéo sont fournies par la caméra au processeur via une interface filaire multimédia haute définition.

5. Masque selon la revendication 2 **caractérisé en ce que** les données vidéo sont fournies par le processeur à l'écran via une interface filaire multimédia haute définition.

6. Masque selon la revendication 2 **caractérisé en ce que** les données vidéo sont fournies au processeur via une interface filaire universelle de bus série.

7. Masque selon l'une quelconque des revendications 2 à 6 **caractérisé en ce que** l'application d'intelligence artificielle traite les données vidéo image par image.

8. Masque selon l'une quelconque des revendications précédentes **caractérisé en ce que** l'algorithme de reconnaissance de formes utilise un histogramme de gradients orientés pour détecter l'arc de soudage.

9. Masque selon l'une quelconque des revendications précédentes **caractérisé en ce que** l'algorithme de reconnaissance de formes utilise un réseau neuronal convolutif pour détecter l'arc de soudage.

10. Masque selon la revendication 1 **caractérisé en ce que** l'algorithme de reconnaissance de formes utilise un modèle pré-entraîné basé sur un réseau neuronal ou un histogramme de gradients orientés, et **en ce que** le modèle comporte un objet correspondant à l'arc de soudage.

11. Masque selon l'une quelconque des revendications 2 à 10 **caractérisé en ce que** l'application d'intelligence artificielle permet l'ajustement de la photosensibilité de la caméra en fonction de la luminosité de l'arc de soudage.

12. Masque selon l'une quelconque des revendications 2 à 11 **caractérisé en ce que** l'application d'intelligence artificielle permet l'ajustement de la photosensibilité de la caméra en fonction de la luminosité de l'environnement ambiant.

13. Masque selon la revendication 1 **caractérisé en ce qu'**il comprend en outre une unité de traitement graphique couplée au processeur pour le traitement des images.

14. Masque selon la revendication 1 **caractérisé en ce que** l'application de traitement d'images utilise un réseau neuronal convolutif pour détecter l'arc de soudage.

15. Masque selon la revendication 1 **caractérisé en ce que** l'application de traitement d'images utilise un modèle pré-entraîné basé sur un réseau neuronal ou un histogramme de gradients orientés, et **en ce que** le modèle comporte un objet correspondant à l'arc de soudage.
